(19) Europäisches Patentamt
European Patent Office
Office européen des brevets

(11) Veröffentlichungsnummer : **0 230 951 B1**

(12) # EUROPÄISCHE PATENTSCHRIFT

(45) Veröffentlichungstag der Patentschrift : **22.05.91 Patentblatt 91/21**

(51) Int. Cl.$^5$ : **C07C 67/54**, C07C 69/67

(21) Anmeldenummer : **87100637.5**

(22) Anmeldetag : **19.01.87**

(54) **Halbacetale von Glyoxylsäureestern sowie Verfahren zur Isolierung von Glyoxylsäureestern.**

(30) Priorität : **25.01.86 DE 3602274**

(43) Veröffentlichungstag der Anmeldung : **05.08.87 Patentblatt 87/32**

(45) Bekanntmachung des Hinweises auf die Patenterteilung : **22.05.91 Patentblatt 91/21**

(84) Benannte Vertragsstaaten : **DE FR**

(56) Entgegenhaltungen :
**EP-A- 0 140 866**
**DE-A- 2 811 480**

(73) Patentinhaber : **HOECHST AKTIENGESELLSCHAFT**
**Postfach 80 03 20**
**W-6230 Frankfurt am Main 80 (DE)**

(72) Erfinder : **Driscoll, Robert Kenneth, Dr.**
**Heilmann-Strasse 43**
**W-6000 Frankfurt am Main (DE)**
Erfinder : **Leupold, Ernst Ingo, Dr.**
**Am Zäunefeld 15**
**W-6392 Neu-Anspach (DE)**
Erfinder : **Schütz, Joachim, Dr.**
**Kurhausstrasse 57A**
**W-6238 Hofheim am Taunus (DE)**

EP 0 230 951 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die vorliegende Erfindung betrifft ein Verfahren zur Isolierung eines Glyoxylsäureesters mit einem $C_1$- bis $C_4$-Alkoholrest aus einem Gemisch, das im wesentlichen einen derartigen Glyoxylsäureester sowie Wasser und oder einen niederen Alkohol mit 1 bis 4 C-Atomen enthält. Neben dem Glyoxylsäureester liegen also im wesentlichen noch Wasser oder ein niederer Alkohol oder beide vor, und zwar teilweise oder vollständig in chemisch gebundener Form, nämlich als Halbacetal, Hydrat bzw. Oligomeres des Glyoxylsäureesters.

Derartige Gemische entstehen beispielsweise bei der Reaktion eines niederen Alkohols ROH mit wäßriger Glyoxylsäure unter simultaner Abdestillation von Wasser, wobei das Halbacetal ROCH(OH)COOR des Glyoxylsäureesters als Hauptprodukt und das entsprechende Vollacetal als Nebenprodukt entsteht. In diesem Fall ist die Estergruppe des Halbacetals vom selben Alkohol ROH abgeleitet wie die Halbacetalgruppe. Ähnlich ist die Situation, wenn das Reaktionsgemisch kondensiert wird, das bei der Oxydehydrierung vor. Glykolsäureester in der Gasphase bei hohem Umsatz entsteht (EP-A2-0 149 456). In diesem Fall enthält das Reaktionsgemisch zusätzlich noch geringe Anteile an unumgesetztem Glykolsäureester.

Weiter entstehen Gemische, die Glyoxylsäureester sowie Wasser und/oder einen niederen Alkohol (in freier oder gebundener Form) enthalten, wenn der Glyoxylsäureester eines bestimmten Alkohols mit einem anderen niederen Alkohol umgesetzt wird. In diesem Fall ist also die Estergruppe des Halbacetals von einem anderen Alkohol abgeleitet als die Halbacetalgruppe.

Die vollständige Gewinnung von reinen Glyoxylsäureestern aus deren Halbacetalen gilt bisher in der Literatur als schwierig, unter anderem wegen der hohen Reaktivität der Verbindungen. Im allgemeinen wird diese Spaltung und Trennung durch Einwirkung von stöchiometrischen Mengen-an $P_2O_5$ und anschließende Destillation erzielt. Diese Verfahrensweise wird z.B. in Oroshnik et al., J. Amer. Chem. Soc. 1941, 63, 3338 beschrieben. Diese Methode ist zwar wirksam, Scheidet aber für ein Großverfahren wegen der hohen Kosten und wegen der damit verbundenen Abfallprobleme aus.

In der Europäischen Patentanmeldung EP-A1-0 140 866 wird ein Verfahren zur Gewinnung reiner Glyoxylsäureester aus Mischungen beschrieben, die Glyoxylsäureester, Wasser und niederen Alkohol sowie verschiedene Verunreinigungen enthalten, aber zusätzlich noch mindestens 1 Mol Glykolsäureester pro Mol Glyoxylsäureester. Solche Mischungen entstehen bei der Oxydehydrierung von Glykolsäureeeter zu Glyoxylsäureester in der Gasphase und anschließender Kondensation, wenn der Umsatz der Oxydehydrierung niedrig ist. Falls notwendig, wird durch Zugabe von Glykolsäureester ein Molverhältnis von Glykolsäureester zu Glyoxylsäureester von mindestens 1 : 1 eingestellt. Mit dieser Methode können jedoch nur etwa 70% des im Einsatzgemisch in freier oder gebundener Form vorliegenden Glyoxylsäureesters gewonnen werden (Beispiel 1).

Es wurde nun gefunden, daß man Glyoxylsäureester im wesentlichen vollständig aus seinem Gemisch mit Wasser und/oder einem niederen Alkohol gewinnen kann, welches wenig oder keinen Glykolsäureester enthält.

Ein Gegenstand der Erfindung ist ein Verfahren zur Gewinnung eines Glyoxylsäureesters mit einem $C_1$- bis $C_4$-Alkoholrest aus einem Gemisch, das im wesentlichen einen derartigen Glyoxylsäureester sowie Wasser und/oder einen $C_1$- bis $C_4$-Alkohol ROH enthält, wobei der Glyoxylsäureester sowie das Wasser und/oder der Alkohol teilweise oder vollständig in chemisch gebundener Form vorliegen, dadurch gekennzeichnet, daß man einen höheren Alkohol R'OH mit einem Siedepunkt oberhalb von 180°C zusetzt und bei einem Druck von höchstens 800 mbar zuerst das Wasser und/oder den Alkohol ROH und anschließend den Glyoxylsäureeater abdestilliert.

Der erste Schritt nach Zugabe des höheren Alkohols R'OH oder während dessen Zugabe, also die Abdestillation von Wasser und/oder Alkohol ROH, und der zweite Schritt, also die Abdestillation des Glyoxylsäureesters, werden vorzugsweise in zwei hintereinandergeschalteten Kolonnen oder Dünnschichtverdampfern kontinuierlich durchgeführt. Man kann aber auch distontinuierlich arbeiten, wobei im allgemeinen eine Kolonne oder ein Dünnschichtverdampfer genügt, worin dann die beiden Schritte der Reihe nach durchgeführt werden. Die kontinuierliche Fahrweise ist bevorzugt, weil die kleineren Verweilzeiten weniger unerwünschte Nebenreaktionen verursachen. Vorzugsweiae verwendet man einen Alkohol R'OH mit einem Siedepunkt über 200°C, weil dann die Gewinnung von Glyoxylsäureester im wesentlichen vollständig ist.

Die Begriffe Glyoxylsäureester, Alkohol und Wasser, in Bezug auf die beim erfindungagemäßen Verfahren eingeaetzte Mischung, sollen, wie bereits oben angedeutet, auch die chemisch gebundenen Formen dieser Substanzen umfassen, wie Glyoxylsäureester-Hydrat, Glyoxylsäureester-Oligomere, Glyoxylsäureester-Halbacetal. Falls zusätzlich geringe Mengen Glykolsäureester anwesend sind, können auch diese als Glyoxylsäureester-Glytolsäureester-Halbacetal vorliegen (da Glykolsäureester noch eine freie Alkoholgruppe hat). Der Gehalt an Glykolaäureeater soll höchstens 0,5 Mol pro Mol Glyoxylsäureester betragen. Besonders geeignet sind Ausgangsgemische die höchstens 0,2 Mol, insbesondere höchstens 0,1 Mol Glykolaäureester pro Mol Glyoxylaäureester enthalten.

Die Alkylreste des Glyoxylsäureesters, des Alkohols ROH und ggf. des Glykolsäureesters haben 1 bis 4

C-Atome und können gleich oder verschieden sein. Vorzugsweise sind sie gleich, insbesondere sind sie sämtlich Methyl. Der Begriff "niederer Alkohol mit 1 bis 4 C-Atomen" soll nicht den Methyl- oder Ethylester der Glykolsäure umfassen (die auch eine freie Alkoholgruppe enthalten und 3 bzw. 4 C-Atome haben), sondern nur Methanol, Ethanol, die Propanole und die Butanole.

Der erste Schritt des erfindungsgemäßen Verfahrens betrifft die destillative Entfernung von Wasser und-/oder niederem Alkohol ROH bei einem Druck von maximal 800 mbar, vorzugsweise etwa 15-530 mbar. Dabei wird der höhere Alkohol R'OH vorher oder gleichzeitig zugesetzt. Es kann beispielsweise in das Reaktionsgemisch eingeleitet werden, das bei der oben erwähnten Oxydehydrierung von Glykolsäureester in der Gasphase entsteht, und die Einleitung kann vor oder nach der Kondensation dieses Reaktionsgemischs erfolgen.

Im allgemeinen sollte das Molverhältnis R'OH zu Glyoxylsäureester bei Einsatz einwertiger Alkohole mindestens 1,0 : 1 betragen. Wenn aber Alkohole mit mehreren OH-Gruppen wie Diethylenglykol oder Triethylenglykol als R'OH verwendet werden, können zufriedenstellende Ergebnisse schon mit Molverhältnissen deutlich unterhalb 1,0 : 1 erzielt werden, nämlich etwa ab 0,5 : 1. Die Wasser- und Alkohol(ROH)Entfernung wird vollständiger mit zunehmendem Molverhältnis.

Im allgemeinen liegt das Molverhältnis also bei einwertigen Alkoholen R'OH im Bereich von etwa 1,0 : 1 bis etwa 4,0 : 1. Bevorzugt ist ein Molverhältnis im Bereich von 1,0 : 1 bis 2,5 : 1.

Bei mehrwertigen Alkoholen wählt man im allgemeinen ein Molverhältnis von 0,5 : 1 bis 4,0 : 1, vorzugsweise 0,5 : 1 bis 2,0 : 1.

Molverhältnisse über 4,0 : 1 sind natürlich auch geeignet, bringen aber keine deutliche Verbesserung in der Wasserund Alkohol-Entfernung und verursachen einen höheren Destillationsaufwand.

Natürlich dürfen die einzusetzenden Alkohole R'OH unter den vorgesehenen Betriebsbedingungen nicht fest werden. Solche mit Schmelzpunkten unterhalb 50°C werden bevorzugt. Insbesondere sind die folgenden Alkohole geeignet, soweit sie einen Siedepunkt oberhalb 180°C, vorzugsweise oberhalb 200°C haben :

a) Geradkettige oder verzweigte Alkanole mit maximal 16 C-Atomen.

b) Polyglykole $HO{+}CH_2CH_2O{+}_nH$ mit n = 2 bis 24

c) Polyglykolmonoether $HO{+}CH_2CH_2O{+}_nR''$ mit n = 2 bis 24 wobei R'' ein geradkettiger oder verzweigter Alkylrest mit maximal 4 C-Atomen ist.

Auch Gemische der genannten Alkohole können verwendet werden. Besonders geeignet sind 1-Decanol, 2-Decanol, Diethylenglykol, Triethylenglykol, Diethylenglykolmonobutglether und Triethylenglykolmonomethylether.

Der Sumpf des 1. Schrittes enthält im wesentlichen Glyoxylsäureester und R'OH, wobei der Glyoxylsäureester zum großen Teil als Halbacetal mit R'OH vorliegt.

Der 2. Schritt des Verfahrens beinhaltet die destillative Gewinnung von reinem Glyoxylsäureester aus diesem Sumpf. Reiner Glyoxylsäureester wird am Kopf gewonnen, während der Alkohol R'OH zurückbleibt. Dieser kann zum 1. Schritt zurückgeführt werden.

Der Druck beim 2. Schritt beträgt wieder maximal 800 mbar, vorzugsweise etwa 15 bis 530 mbar. Der Glyoxylsäureester kann im Prinzip vollständig vom Alkohol R'OH abdestilliert werden. Dies erfordert jedoch relativ hohe Temperaturen, was erhöhte Zersetzungsgetahr bedeutet. Daher destilliert man vorzugsweise den Glyoxylsäureester nicht ganz vollständig ab. Der zuruckbleibende Sumpf enthält dann neben dem Alkohol R'OH noch kleine Mengen Glyoxylsäureester. Dieser Sumpf kann erneut in das erfindungsgemäße Verfahren eingesetzt werden.

Ein weiterer Gegenstand der Erfindung sind Halbacetale von Glyoxylsäureestern der allgemeinen Formel R'''OCH(OH)COOR*, wobei R* ein geradkettiger oder verzweigter Alkylrest mit 1 bis 4 C-Atomen, vorzugsweise der Methylrest ist, und wobei R''' eine der folgenden Strukturen hat :

a) ein geradkettiger oder verzweigter Alkylrest mit 9 bis 16 C-Atomen, vorzugsweise 1-Decyl, 2-Decyl oder 1-Dodecyl.

b) ${+}CH_2CH_2O{+}_nH$ mit n = 2 bis 24, vorzugsweise n = 2 oder 3.

c) ${+}CH_2CH_2O{+}_nR''$ mit n = 2 bis 24, vorzugsweise n = 2 oder 3, wobei R'' ein geradkettiger oder verzweigter Alkylrest mit maximal 4 C-Atomen ist.

Aus diesen Halbacetalen kann man die freien Glyoxylsäureester herstellen, indem man die Halbacetale erwärmt und destilliert.

Die freien Glyoxylsäureester sind aufgrund ihrer hohen Reaktivität wertvolle Ausgangs- und Zwischenprodukte für eine Reihe von Synthesen pharmazeutisch wirksamer Verbindungen, wie z.B. Allantoin, substituierter Glycine oder Alkaloide (wie z.B. Tetrahydroisochinolin-Alkaloide).

Neuerdings wird der Einsatz von Glyoxylsäureester zur Herstellung polymerer biologisch abbaubarer Phosphatersatzstoffe für Waschmittel diskutiert.

Die nachfolgenden Beispiele sollen die Erfindung näher erläutern.

Beispiel 1

1 mol (88 g) Glyoxylsäuremethylester wurde bei 130 mbar über eine Claisen-Brücke in eine Vorlage mit Rührer destilliert, die 1 mol des jeweiligen Alkohols-R'OH enthielt. Dieses Gemisch wurde im Sumpf einer Destillationskolonne (Länge 450 mm, Durchmesser 20 mm, Füllung : Braunschweiger Glaswendeln, Rücklaufverhältnis 4 : 1) vorgelegt und bei konstantem Druck destilliert. Während der Destillation wurden häufig Proben am Kolonnenkopf entnommen und gaschromatographisch oder mit klassischen Methoden analysiert. Die ersten Fraktionen enthielten fast ausschließlich Glyoxylsäuremethylester. Die Destillationen wurden fortgesetzt, bis Fraktionen, die kleine Mengen an jeweiligem R'OH enthielten, am Kopf festgestellt wurden. Die Ergebnisse von Versuchen bei verschiedenen Betriebsdrucken und mit verschiedenen Alkoholen R'OH sind in der Tabelle 1 wiedergegeben. Destillationen bei 80 mbar ergaben hohe Reinheiten (um 97,5%) ; die Verunreinigungen waren im wesentlichen Wasser, Methanol und R'OH. Aus den Ergebnissen ist ersichtlich, daß die Menge an isoliertem Glyoxylsäuremethylester mit zunehmendem R'OH-Siedepunkt deutlich ansteigt. Aus dem Vergleichsversuch 1a mit Glykolsäuremethylester als R'OH (gemäß EP-A1-0 140 866) lassen sich nur wesentlich geringere Ausbeuten als nach dem erfindungsgemäßen Verfahren erzielen. Besonders gute Ergebnisse wurden mit den bevorzugten Alkoholen in den Versuchen (1e) bis (1j) erreicht.

Tabelle 1

| | Alkohol R'OH | Siedepunkt bei Normaldruck | Destillationsdruck (mbar) | $OHCCOOCH_3$-Ausbeute (%) | $OHCCOOCH_3$-Gehalt des Destillats (Gew.-%) |
|---|---|---|---|---|---|
| a | Glykolsäuremethylester (Vergleich) | 151°C | 80 | 32,2 | 97,2 |
| b | 2-Ethylhexanol | 184°C | 333<br>80 | 56,1<br>25,7 | 95,1<br>96,7 |
| c | Diethylenglykolmonomethylether | 193°C | 27 | 71,3 | 97,3 |
| d | n-Octanol | 194°C | 80 | 63,1 | 97,7 |
| e | Diethylenglykolmonobutylether | 228°C | 80 | 96,2 | 96,9 |
| f | n-Decanol | 230°C | 80 | 97,4 | 98,3 |
| g | Diethylenglykol | 245°C | 80 | 98,1 | 98,4 |
| h | Triethylenglykolmonomethylether | 249°C | 80 | 96,2 | 97,1 |
| i | Triethylenglykol | 276°C | 27 | 97,0 | 98,1 |
| j | Triethylenglykol-mono-n-butylether | 282°C | 80 | 96,0 | 97,6 |

Beispiel 2

Glykolsäuremethylester wurde nach dem in DE-OS 3 417 649 beschriebenen Verfahren oxidiert und der Reaktoraustrag wurde kondensiert. Pro 100 g Kondensat wurden 117 g Diethylenglykol zugegeben, wobei ein Gemisch mit folgender Zusammensetzung (in Gew.-%) entstand : 36,1% Glyoxyläuremethylester, 7,8% Wasser, 1,3% Methanol, 53,9% Diethylenglykol und 0,9% Sonstige. 80 g pro Stunde dieses Gemisches wurden auf etwa 70°C vorgeheizt und in den Mittelteil einer kontinuierlich betriebenen Destillationskolonne (Länge 100 cm, Durchmesser 2 cm, Füllkörper : Braunschweiger Glaswendeln) eingeleitet. Die Schütthöhe der Glaswendeln betrug im Abtriebsteil 60 cm, im Verstärkerteil 30 cm. Die Kolonne wurde bei 40 mbar betrieben, das Rücklaufverhältnis betrug 2 : 1. Während des Versuchs betrug die Sumpftemperatur etwa 115°C. Nach der Einstellung des Gleichgewichts fielen 72,6 g Produkt pro Stunde im Sumpf mit folgender Zusammensetzung

(in Gew.-%) an : 39,7% Glyoxylsäuremethylester, 0,34% Wasser, 0,14% Methanol, 59,4% Diethylenglykol und 0,42% Sonstige. Wasser und Methanol wurden also weitgehend entfernt. Die Kolonne wurde gereinigt und für den 2. Schritt verwendet.

Das Sumpfprodukt des 1. Schritts diente als Einsatz für den 2. Schritt. 66,0 g pro Stunde dieses Gemisches wurden auf etwa 70°C vorgeheizt und an derselben Stelle wie im 1. Schritt in den Mittelteil der Destillationskolonne eingeleitet. Die Kolonne wurde bei 80 mbar betrieben, das Rücklaufverhältnis betrug 2 :1. Die Sumpf- und Kopftemperaturen betrugen während des Versuches etwa 167°C bzw. 51°C. Nach der Einstellung des Gleichgewichts wurden 25,5 g pro Stunde Kopfprodukt mit folgender Zusammensetzung (in Gew.-%) gewonnen : 98,8% Glyoxylsäuremethylester, 0,67% Wasser, 0,1% Methanol und 0,43% Sonstige. Außerdem sind 40,4 g pro Stunde Sumpfprodukt mit folgender Zusammensetzung (in Gew.-%) angefallen : 2,23% Glyoxylsäuremethylester, 0,03% Wasser, 0,17% Methanol, 97,0% Diglykol und 0,57% Sonstige.

Beispiel 3

Die im Beispiel 1 beschriebene Destillationskolonne wurde hier erneut verwendet. Das Einsatzgemisch hatte folgende Zusammensetzung (in Gew.-%) : 29,5% Glyoxylsäuremethylester, 1,5% Glykolsäuremethylester und 69,0% Triethylenglykolmono-n-butylether. 250 g dieses Gemisches wurden in der Destillationsblase vorgelegt und mit einem Rücklaufverhältnis von 4 : 1 und 80 mbar Druck destilliert. Die Kopftemperatur betrug etwa 51°C während der Abnahme der Hauptfraktion, die aus 68,8 g an 97,7 %igem Glyoxylsäuremethylester bestand. Der restliche Glyoxylsäuremethylester wurde als Mischfraktionen zusammen mit Glykolsäuremethylester und Triglykolbutylether isoliert.

Beispiel 4

200,0 g eines Gemisches folgender Zusammensetzung (in Gew.-%) wurden in der Blase einer Destillationskolonne (Länge 100 cm, Durchmesser 2 cm, Füllkörper : Braunschweiger Glaswendeln) vorgelegt : 95,2% $CH_3OCH(OH)\text{-}COOCH_3$, 2,1%, $(CH_3O)_2CH\text{-}COOCH_3$, 1,7% OCH-COOH, 0,5 % $CH_3OH$ und 0,5% Wasser. Das Gemisch wurde bei 60 mbar und einem Rücklaufverhältnis von 3 : 1 destilliert und gleichzeitig wurden 180 g Diethylenglykol (120 g/h) im obersten Drittel der Kolonne eingeleitet. Nach einem 1 1/2 stündigen Versuch enthielt der Sumpf 327,4 g eines Gemisches folgender Zusammensetzung (in Gew.-%) : 42,6% $CHO\text{-}COOCH_3$, 1,3% $(CH_3O)_2CH\text{-}COOCH_3$, 1,0% OCH-COOH, 0,06% Wasser und 55,0% Diglykol, wobei Glyoxylsäuremethylester überwiegend in der Halbacetal-Form mit Diglykol vorlag. Am Kopf wurden 52,6 g eines Gemisches mit folgender Zusammensetzung erhalten : 98,5 Gew.-% Methanol und 1,5 Gew.-% Wasser. Das Sumpfprodukt enthielt im wesentlichen Glyoxylsäuremethylester und Diglykol.

Beispiel 5

0,74 mol (65 g) Glyoxylsäuremethylester wurden bei 100 mbar über eine Claisen-Brücke in eine Vorlage mit Rührer destilliert, die 0,74 mol (78,4 g) Diethylenglykol enthielt. Das entstande Halbacetal wurde durch sein $C^{13}$-NMRSpektrum (in Aceton $d_6$ aufgenommen) charakterisiert.

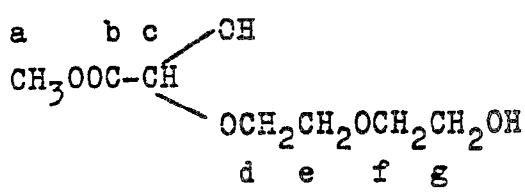

| C-Atom | Chemische Verschiebung (ppm) | relative Intensität |
|---|---|---|
| a | 52,4 | 1 |
| b | 169,7 | 1 |
| c | 93,3 | 1 |
| d | 67,2 | 1 |
| e | 70,5 | 1 |
| f | 72,7 | 1 |
| g | 61,5 | 1 |

Keine anderen Peaks sind im Spektrum vorhanden. Zahl der Peaks, Peaklagen sowie relative Intensitäten korrelieren mit der Halbacetal-Struktur. Diglykol würde zwei Peaks, nämlich bei 61,4 und 72,6 ppm zeigen, während das Halbacetal vier O-$CH_2$-Signale (bei 61,5, 72,7, 67,2 und 70,5 ppm) aufweist (d bis g). Das Signal bei 93,3 ppm ist charakteristisch für ein Halbacetal-C-atom mit folgender Struktur :

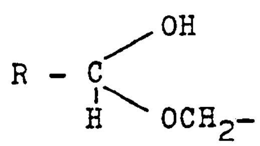

wobei R = $COOCH_3$

Peaks, die für unreagierten Glyoxylsäuremethylester charakteritisch sind, waren nicht vorhanden.

<u>Beispiel 6</u>

0,34 mol (29,9 g) Glyoxylsäuremethylester wurden bei 130 mbar über eine Claisen-Brücke in eine Vorlage mit Rührer destilliert, die 0,34 mol (55,1 g) Diethylenglygtolmono-n-butylether enthielt. Das entstandene Halbacetal wurde durch sein $C^{13}$-NMR-Spettrum (in Aceton $d_6$ aufgenommen) charakterisiert :

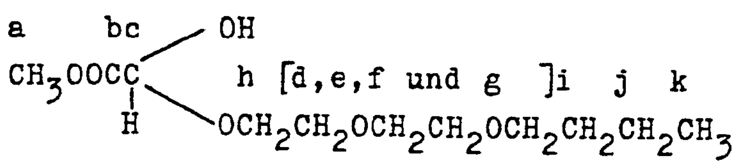

| C-Atom | Chemische Verschiebung (ppm) | Signale | relative Intensität |
|---|---|---|---|
| a | 52,2 | 1 | 1 |
| b | 169,4 | 1 | 1 |
| c | 93,4 | 1 | 1 |
| d+e+f+g | 70,3 bis 71,0 | 4 | Σ = 4 |
| h | 67,3 | 1 | 1 |
| i | 32,1 | 1 | 1 |
| j | 19,6 | 1 | 1 |
| k | 14,0 | 1 | 1 |

Keine anderen Peaks sind im Spettrum vorhanden. Zahl der Peaks, Peaklagen sowie relative Intensitäten korrelieren mit der Halbacetal-Struktur. Das Signal bei 93,4 ppm ist charakteristisch für ein Halbacetal-C-Atom mit folgender Struktur :

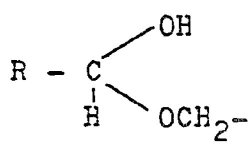

wobei R = $COOCH_3$

Beispiel 7

0,35 mol (30,8 g) Glyoxylsäuremethylester wurden bei 100 mbar über eine Claisen-Brücke in eine Vorlage mit Rührer destilliert, die 0,35 mol (55,3 g) n-Decanol enthielt. Das entstandene Halbacetal wurde durch sein $C^{13}$-NMR-Spektrum (in Aceton $d_6$ aufgenommen) charakterisiert :

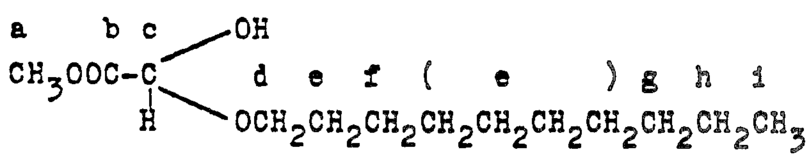

| C-Atom | Chemische Verschiebung (ppm) | Signale | relative Intensität |
|---|---|---|---|
| a | 52,1 | 1 | 1 |
| b | 170,1 | 1 | 1 |
| c | 93,3 | 1 | 1 |
| d | 68,1 | 1 | 1 |
| e | 29,8 - 30,2 | 5 | Σ = 5 |
| f | 26,6 | 1 | 1 |
| g | 32,4 | 1 | 1 |
| h | 23,1 | 1 | 1 |
| i | 14,2 | 1 | 1 |

Keine anderen Peaks sind im Spektrum vorhanden. Zahl der Peaks, Peaklagen sowie relative Intensitäten korrelieren mit der Halbacetal-Struktur.

## Ansprüche

1. Verfahren zur Gewinnung eines Glyoxylsäureesters mit einem $C_1$- bis $C_4$-alkoholrest aus einem Gemisch, das im wesentlichen einen derartigen Glyoxylsäureester sowie Wasser und/oder einen $C_1$- bis $C_4$-Alkohol ROH enthält, wobei der Glyoxylsäureester sowie das Wasser und/oder der Alkohol teilweise oder vollständig in chemisch gebundener Form vorliegen, dadurch gekennzeichnet, daß man einen höheren Alkohol R'OH mit einem Siedepunkt über 180°C zusetzt und bei einem Druck von höchstens 800 mbar zuerst das Wasser und/oder den Alkohol ROH und anschließend den Glyoxylsäureester abdestilliert.

2. Verfahren nach Anspruch 1, dadurch gekennzeichnet, daß man einen höheren Alkohol R'OH mit einem Siedepunkt über 200°C verwendet.

3. Verfahren nach Anspruch 1 oder 2, dadurch gekennzeichnet, daß man einen höheren Alkohol R'OH verwendet, der zu einer der drei folgenden Klassen gehört :

a) Geradkettige oder verzweigte Alkanole mit maximal 16 C-Atomen.

b) Polyglykole $HO(CH_2CH_2O)_nH$ mit n = 2 bis 24.

c) Polyglykolmonoether $HO(CH_2CH_2O)_n R''$ mit n = 2 bis 24, wobei R" ein geradkettiger oder verzweigter Alkylrest mit maximal 4 C-Atomen ist.

4. Verfahren nach einem der Ansprüche 1 bis 3, dadurch gekennzeichnet, daß man bei einem Druck von etwa 15 bis etwa 530 mbar arbeitet.

5. Verfahren nach einem der Ansprüche 1 bis 4, dadurch gekennzeichnet, daß als Glyoxylsäureester der Methylester und als niedere Alkohol ROH Methanol vorliegt.

6. Halbacetale von Glyoxylsäureestern der allgemeinen Formel R"'OCH(OH)COOR*, wobei R* ein geradkettiger oder verzweigter Alkylrest mit 1 bis 4 C-Atomen, vorzugsweise der Methylrest ist, und wobei R"' eine der folgenden Strukturen hat :

a) ein geradkettiger oder verzweigter Alkylrest mit 9 bis 16 C-Atomen, vorzugsweise 1-Decyl, 2-Decyl oder 1-Dodecyl.

b) $(CH_2CH_2O)_nH$ mit n = 2 bis 24, vorzugsweise n = 2 oder 3.

c) $(CH_2CH_2O)_nR''$ mit n = 2 bis 24, vorzugsweise n = 2 oder 3, wobei R" ein geradkettiger oder verweigter Alkylrest mit maximal 4 C-Atomen ist.

## Claims

1. A process for obtaining a glyoxylic ester having a $C_1$ to $C_4$ alcohol residue from a mixture which essentially contains a glyoxylicester of this type plus water and/or a $C_1$ to $C_4$ alcohol ROH, the glyoxylic ester and the water and/or the alcohol being present partly or completely in chemically bonded form, which comprises addition of a higher alcohol R'OH having a boiling point above 180°C, and distillation out, under a pressure not exceeding

800 mbar, of first the water and/or the alcohol ROH and then the glyoxylic ester.

2. The process as claimed in claim 1, wherein a higher alcohol R'OH having a boiling point above 200°C is used.

3. The process as claimed in claim 1 or 2, wherein use is made of a higher alcohol R'OH which belongs to one of the three following classes :

a) Straight-chain or branched alkanols having a maximum of 16 carbon atoms.

b) Polyglycols $HO(CH_2CH_2O)_nH$ with n = 2 to 24.

c) Polyglycol monoethers $HO(CH_2CH_2O)_nR''$ with n = 2 to 24, R'' being a straight-chain or branched alkyl radical having a maximum of 4 carbon atoms.

4. The process as claimed in one of claims 1 to 3, witch is operated under a pressure of about 15 to about 530 mbar.

5. The process as claimed in one of claims 1 to 4, wherein the glyoxylic ester is in the form of the methyl ester and the lower alcohol ROH is in the form of methanol.

6. Hemiacetals of glyoxylic esters of the formula R'''OCH(OH)COOR*, R* being a straight-chain or branched alkyl radical having 1 to 4 carbon atoms, preferably the methyl radical, and R''' having one of the following structures :

a) a straight-chain or branched alkyl radical having 9 to 16 carbon atoms, preferably 1-decyl, 2-decyl or 1-dodecyl.

b) $(CH_2CH_2O)_nH$ with n = 2 to 24, preferably n = 2 or 3.

c) $(CH_2CH_2O)_nR''$ with n = 2 to 24, preferably n = 2 or 3, R'' being a straight-chain or branched alkyl radical having a maximum of 4 carbon atoms.

## Revendications

1. Procédé pour isoler un ester de l'acide glyoxylique qui contient de 1 à 4 atomes de carbone dans sa partie alcool à partir d'un mélange renfermant essentiellement un tel ester glyoxylique ainsi que de l'eau et/ou un alcool ROH en $C_1$-$C_4$, l'ester glyoxylique ainsi que l'eau et/ou l'alcool étant partiellement ou totalement à l'état chimiquement lié, procédé caractérisé en ce qu'on ajoute un alcool supérieur R'OH dont le point d'ébullition est supérieur à 180°C et on chasse par distillation, sous une pression d'au plus 800 mbar, d'abord l'eau et/ou l'alcool ROH, puis l'ester de l'acide glyoxylique.

2. Procédé selon la revendication 1 caractérisé en ce qu'on utilise un alcool supérieur R'OH qui a un point d'ébullition supérieur à 200°C.

3. Procédé selon l'une des revendications 1 et 2, caractérisé en ce qu'on utilise un alcool supérieur R'OH qui appartient à l'une des trois catégories suivantes :

a) celle des alcanols linéaires ou ramifiés qui contiennent au plus 16 atomes de carbone,

b) celle des polyglycols $HO(CH_2CH_2O)H$ dans lesquels n désigne un nombre de 2 à 24, et

c) celle des mono-éthers de polyglycols $HO(CH_2CH_2O)nR''$ dans lesquels n désigne un nombre de 2 à 24 et R'' représente un radical alkyle linéaire ou ramifié qui contient au plus 4 atomes de carbone.

4. Procédé selon l'une quelconque des revendications 1 à 3, caractérisé en ce qu'on opère sous une pression d'environ 15 à environ 530 mbar.

5. Procédé selon l'une quelconque des revendications 1 à 4, caractérisé en ce que l'ester de l'acide glyoxylique est l'ester méthylique, et l'alcool inférieur ROH est le méthanol.

6. Hémi-acétals d'esters de l'acide glyoxylique répondant à la formule générale :

R'''OCH(OH)COOR*

dans laquelle R* représente un alkyle, linéaire ou ramifié, qui contient de 1 à 4 atomes de carbone, de préférence un radical méthyle, et R''' représente :

a) un alkyle linéaire ou ramifié qui contient de 9 à 16 atomes de carbone, de préférence un radical décyle-1, méthyl-1 nonyle ou dodécyle-1,

b) un radical $(CH_2CH_2O)nH$ dans lequel n désigne un nombre de 2 à 24, de préférence le nombre 2 ou le nombre 3, ou

c) un radical $(CH_2CH_2O)nR''$ dans lequel n désigne un nombre de 2 à 24, de préférence le nombre 2 ou le nombre 3, et R'' représente un alkyle linéaire ou ramifié qui contient au plus 4 atomes de carbone.